# NEUE EUROPÄISCHE PATENTSCHRIFT

(11) **EP 3 941 652 B2**
(45) Veröffentlichungstag und Bekanntmachung des Hinweises auf die Entscheidung über den Einspruch: **08.07.2026**
(45) Hinweis auf die Patenterteilung: 16.11.2022
(21) Anmeldenummer: 20711576.7
(22) Anmeldetag: 12.03.2020
(51) Int. Cl.: B08B 15/02

(54) **ANLAGE ZUM HANDHABEN VON EMPFINDLICHEN PRODUKTEN, INSBESONDERE VERPACKUNGSANLAGE**
PLANT FOR HANDLING SENSITIVE PRODUCTS, IN PARTICULAR PACKAGING PLANT
DISPOSITIF DE MANIPULATION DE PRODUITS SENSIBLES, NOTAMMENT DISPOSITIF DE CONDITIONNEMENT

(30) Priorität: 18.05.2019 DE 102019207280
(43) Veröffentlichungstag der Anmeldung: 26.01.2022
(73) Patentinhaber: Syntegon Technology GmbH, 70372 Stuttgart (DE)
(72) Erfinder: METZGER, Simon, 89297 Roggenburg (DE); MARTIN, David, Durham DH6 4HA (GB); HAAG, Heiko, 91550 Dinkelsbuehl (DE); GRAF, Sebastian, 74589 Satteldorf (DE); WOOD, Nigel, Durham DL12 8SA (GB)
(74) Vertreter: DREISS Patentanwälte PartG mbB
(86) Internationale Anmeldenummer: PCT/EP2020/056720
(87) Internationale Veröffentlichungsnummer: WO 2020/233854

(56) Entgegenhaltungen:
- EP-A1- 2 733 196
- EP-A2- 1 460 126
- WO-A1-2009/147252
- WO-A1-2017/072591
- WO-A2-2018/050889
- DE-A1- 19 947 786

## Beschreibung

### Stand der Technik

Die vorliegende Erfindung betrifft eine Anlage zum Füllen und Verschließen von Behältnissen für Pharmazeutika, nämlich Medikamente.

Bei der Handhabung von pharmazeutischen Produkten müssen beim Befüllen und Verschließen von Behältnissen, in welche derartige pharmazeutische Produkte abgefüllt werden, erhöhte Reinheitsanforderungen erfüllt werden. Eine derartige Verpackungsanlage ist beispielsweise aus der DE 199 47 786 A1 bekannt. Hierbei umfasst die Verpackungsanlage einen als Isolator ausgebildeten Reinraum mit höchsten Anforderungen. Es ist weiterhin bekannt, dass die Luft in derartigen Reinräumen überwacht wird, um eine mikrobiologische Kontamination des abzufüllenden Produkts zu vermeiden. Hierzu sind in dem Reinraum Petrischalen mit einem entsprechenden Nährmedium angeordnet. Die Handhabung derartiger Petrischalen erfolgt hierbei manuell durch einen Handschuheingriff im Isolator. Gegebenenfalls erfolgt auch noch ein händisches Beschriften der Petrischale, um die Petrischale einer abgefüllten Charge des Produkts zuordnen zu können. Durch den manuellen Eingriff in die Verpackungsanlage erhöht sich jedoch das Risiko, dass ein Fehler beim manuellen Handling auftreten kann. Auch kann das Petrischalenhandling nicht reproduzierbar durch den Faktor Mensch vorgenommen werden. Auftretende menschliche Fehler sind beispielsweise eine zu frühe oder zu späte Öffnung der Petrischale oder eine falsche Positionierung der Petrischale innerhalb des Reinraums. Weitere Fehlerquellen liegen in einer falschen oder unvollständigen Beschriftung. Es wäre daher wünschenswert, eine verbesserte Verpackungsanlage zu haben.

EP 1 460 126 A2 und WO 2018 / 050 889 A2 zeigen jeweils eine Anlage mit Merkmalen des Anspruchs 1.

### Offenbarung der Erfindung

Die erfindungsgemäße Anlage zum Handhaben von empfindlichen Produkten mit den Merkmalen des Anspruchs 1 weist demgegenüber den Vorteil auf, dass eine sichere und einfache Handhabung durch Automatisierung möglich ist. Dabei sind empfindliche Produkte im Sinne der Erfindung Medikamente. Hierbei können menschliche Fehler bei der mikrobiologischen Luftüberwachung innerhalb der Anlage vermieden werden. Manuelle Eingriffe durch einen Menschen von außerhalb der Anlage sind nicht mehr notwendig. Dadurch ergibt sich, wenn beispielsweise Pharmazeutika abgefüllt werden, eine signifikante Reduzierung eines pharmazeutischen Risikos für den Gesamtprozess innerhalb der Anlage. Auch kann möglichen Verletzungen eines Nutzers, welche bei einem Handschuheingriff in das Innere der Anlage bestehen, ausgeschlossen werden.

Dies wird erfindungsgemäß dadurch erreicht, dass die Anlage einen abgeschlossenen Raum, in welchem die empfindlichen Produkte gehandhabt werden, und eine Einrichtung zur Überwachung der Luft im abgeschlossenen Raum umfasst. Die Einrichtung zur Überwachung der Luft umfasst dabei eine Messstelle und wenigstens eine Petrischale. Die Petrischale ist im Inneren des abgeschlossenen Raums angeordnet und umfasst eine untere Aufnahme (Schale) und einen Deckel. Weiterhin umfasst die Anlage eine automatische Handhabungseinrichtung, wobei die Handhabungseinrichtung eingerichtet ist, automatisiert den Deckel von der unteren Aufnahme der Petrischale zu entfernen und aufzusetzen. In der Petrischale ist ein Nährboden oder dergleichen vorgesehen, um eine mikrobiologische Luftüberwachung im abgeschlossenen Raum zu ermöglichen. Dazu wird nun automatisiert mittels der automatischen Handhabungseinrichtung die Petrischale verschlossen zur Messstelle gebracht, der Deckel von der Petrischale entfernt und an einem vorbestimmten Lagerort abgelegt. Nach Ablauf einer vorbestimmten Zeit wird dann der Deckel wieder aufgenommen, auf die untere Aufnahme der Petrischale aufgesetzt und diese z.B. zu einem Lagerplatz in einem Magazin gebracht. Gegebenenfalls wird automatisch eine neue Petrischale entnommen und an der Messstelle positioniert und der Deckel der neuen Petrischale abgenommen und eine zweite Messung durchgeführt. Somit kann erfindungsgemäß die mikrobiologische Luftüberwachung vollständig automatisiert im Inneren der Anlage ausgeführt werden, ohne dass ein Nutzer von außen durch einen Handschuheingriff oder dergleichen ein Petrischalenhandling ausführen muss.

Erfindungsgemäß ist die automatische Handhabungseinrichtung eingerichtet Transportschritte für Behältnisse auszuführen. Beispielsweise kann ein Transport von Behältnissen, in welche ein Produkt abgefüllt werden soll, im Inneren des abgeschlossenen Raumes zwischen einer Füllstation und einer Verschließstation für das Behältnis, ausgeführt werden. Alternativ oder zusätzlich kann die automatische Handhabungseinrichtung auch leere und/oder gefüllte Behältnisse von einem Lagerort oder zu einem Lagerort transportieren.

Die Unteransprüche zeigen bevorzugte Weiterbildungen der Erfindung.

Vorzugsweise umfasst die Anlage eine Vielzahl von Petrischalen. Die Vielzahl von Petrischalen sind vorzugsweise in einem Luftkeimsammler mit einem Sammelkopf und einem Magazin für die Petrischalen vorgesehen. Die automatische Handhabungseinrichtung kann hierbei bevorzugt auch den Sammelkopf des Luftkeimsammlers automatisiert abnehmen und aufsetzen. Hierdurch kann ein Automatisierungsgrad weiter erhöht werden.

Besonders bevorzugt umfasst die Anlage ferner eine Füllstation zum Abfüllen der empfindlichen Produkte in Behältnisse und eine Verschließstation zum Verschließen der befüllten Behältnisse. Die automatische Handhabungseinrichtung ist dabei besonders bevorzugt eingerichtet, die Behältnisse von der Füllstation zur Verschließstation zu transportieren. Dabei können die Behältnisse einzeln transportiert werden oder alternativ werden gleichzeitig mehrere Behältnisse transportiert. Hierbei kann eine zusätzliche Formateinrichtung vorgesehen werden, um mehrere Behältnisse gleichzeitig aufzunehmen.

Die automatische Handhabungseinrichtung ist besonders bevorzugt ein Roboter. Der Roboter ist vorzugsweise ein 6-Achs-Roboter. Der Roboter weist weiter bevorzugt einen Greifer mit wenigstens zwei Greifbacken auf. Behältnisse oder die Petrischale können dabei zwischen den beiden Greifbacken geklemmt werden. Weiter bevorzugt kann der Roboter den Greifer wechseln, so dass unterschiedliche Greifbacken am Roboter befestigbar sind. Dies kann beispielsweise dann notwendig werden, wenn die automatische Handhabungseinrichtung sowohl für die Luftüberwachung mittels der Petrischalen als auch zum Transport von Behältnissen eingesetzt wird.

Gemäß einer weiteren bevorzugten Ausgestaltung der vorliegenden Erfindung umfasst die Anlage ferner eine Identifikationseinrichtung. Die Identifikationseinrichtung ist dabei eingerichtet, Petrischalen zu identifizieren und eine Messzeit, bei welcher die Petrischale geöffnet war, um eine mikrobiologische Luftüberwachung durchzuführen, dieser Petrischale zuzuordnen.

Dabei weist vorzugsweise jede Petrischale ein individuelles, optisches Erkennungszeichen auf. Dies kann beispielsweise ein Barcode oder eine Identifikationsnummer oder dergleichen sein. Ferner weist die Identifikationseinrichtung eine optische Erkennungseinrichtung auf. Dies kann beispielsweise ein Scanner oder eine Kamera oder dergleichen sein.

Besonders bevorzugt ist der abgeschlossene Raum der Anlage derart ausgebildet, dass die Anlage eine Isolatoranlage oder eine RABS-Anlage (restricted-access barrier system). Somit handelt es sich bei der erfindungsgemäßen Anlage um eine Reinraumanlage mit erhöhten Anforderungen an die Luftqualität. Dies ist beispielsweise bei der Handhabung von Pharmazeutika oder Feinchemikalien oder dergleichen notwendig.

### Kurze Beschreibung der Zeichnungen

Nachfolgend wird eine Anlage zum Handhaben von empfindlichen Produkten gemäß einem bevorzugten Ausführungsbeispiel der Erfindung im Detail beschrieben. In der Zeichnung ist:
- Figur 1: eine schematische, perspektivische Ansicht einer Anlage gemäß einem bevorzugten Ausführungsbeispiel der Erfindung und
- Figuren 2 bis 4: schematische Darstellungen, welche die automatisierte Handhabung innerhalb der Anlage zur mikrobiologischen Luftüberwachung zeigen.

### Bevorzugte Ausführungsformen der Erfindung

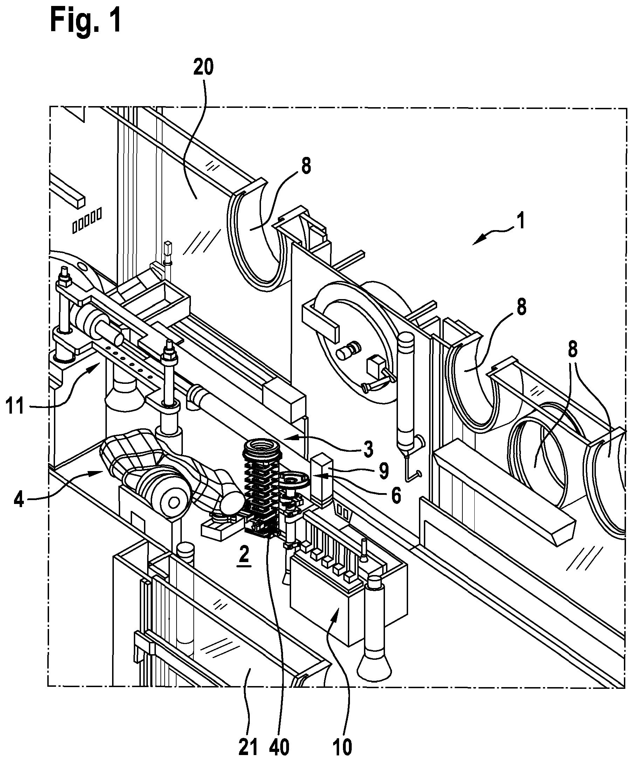

Nachfolgend wird unter Bezugnahme auf die Figuren 1 bis 4 eine Füll- und Verpackungsanlage gemäß einem ersten bevorzugten Ausführungsbeispiel der Erfindung im Detail beschrieben.

Wie aus der schematischen Ansicht von Figur 1 ersichtlich ist, umfasst die Anlage 1 einen abgeschlossenen Raum 2, welcher durch Raumwände 20, 21 von einer Umgebung abgeschlossen ist. In der Raumwand 20 sind hierbei mehrere Handeingriffe 8 vorgesehen, um einen Handschuheingriff (nicht dargestellt) in den abgeschlossenen Raum 2 zu ermöglichen.

Die Anlage 1 ist eine Füll- und Verschließanlage für pharmazeutische Produkte, nämlich Medikamente, und umfasst eine Füllstation 10 und eine Verschließstation 11. Diese sind in Figur 1 nur schematisch dargestellt.

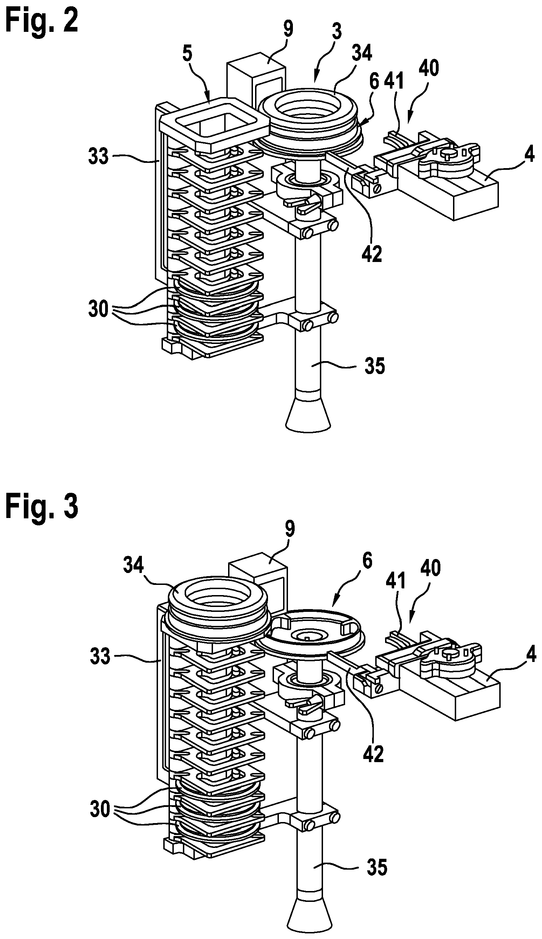

Weiterhin ist zur mikrobiologischen Luftüberwachung im abgeschlossenen Raum 2 eine Einrichtung 3 zur Luftüberwachung vorgesehen. Die Einrichtung 3 ist im Detail aus den Figuren 2 bis 4 ersichtlich.

Die Einrichtung 3 zur Luftüberwachung umfasst dabei ein Magazin 33 mit einer Vielzahl von Petrischalen 30. In diesem Ausführungsbeispiel sind genau drei Petrischalen 30 vorgesehen.

Jede Petrischale 30 umfasst eine untere Aufnahme 31 (Schale) sowie einen Deckel 32. In der unteren Aufnahme 31 ist ein Nährmedium oder dergleichen eingebracht. Während des Abfüllens und des Verschließens der pharmazeutischen Produkte wird die Petrischale geöffnet, so dass sich eventuell im abgeschlossenen Raum 2 befindliche Keime in der Petrischale absetzen.

Nach einem erfolgten Abfüllen und Verschließen der Behältnisse kann diese Petrischale dann aus der Anlage 1 entnommen werden und einer weiteren Untersuchung zugeführt werden. Erst nachdem festgestellt wurde, dass sich keine Keime in der während der Abfüllung geöffneten Petrischale befinden, wird dann die entsprechend abgefüllte Charge von Pharmazeutika freigegeben.

Wie aus den Figuren 2 bis 4 ersichtlich ist, umfasst die Einrichtung 3 zur Luftüberwachung hierzu ein vertikales Magazin 33, in welchem Petrischalen 30 übereinander gelagert werden können. Neben dem Magazin 33 befindet sich ein sogenannter Sammelkopf 34 eines Luftkeimsammlers, in welchem eine geöffnete Petrischale platziert wird, um die Luftüberwachung durchzuführen. Eine Messstelle 6 ist somit im Sammelkopf 34 vorgesehen.

Die Einrichtung 3 zur Luftüberwachung umfasst neben dem Sammelkopf 34 ferner ein Luftaustrittsrohr 35, so dass Luft von oben in den Sammelkopf 34 auf die darin platzierte geöffnete Petrischale strömt und über das Luftaustrittsrohr 35 abgegeben wird.

Ferner umfasst die Anlage 1 eine Identifikationseinrichtung 9, beispielsweise einen Scanner oder eine Kamera, welche zur Identifizierung der jeweils benutzten Petrischale 30, welche gerade zur Luftüberwachung eingesetzt wird, dient. Hierzu weisen die Petrischalen 30 beispielsweise Barcodes oder Identifikationsnummern oder dergleichen auf, um sicher mittels der Identifikationseinrichtung 9 erkannt zu werden.

Abhängig von der Länge des Füll- und Verschließvorgangs müssen mehrere Petrischalen 30 zur Luftüberwachung verwendet werden.

Hierzu umfasst die Anlage 1 ferner eine automatische Handhabungseinrichtung 4, welche in diesem Ausführungsbeispiel ein 6-achsiger Roboter ist. Die automatische Handhabungseinrichtung 4 führt automatisch die mikrobiologische Luftüberwachung mittels der Petrischalen 30 durch.

In der in Figur 2 gezeigten Ausgangsposition sind in diesem Ausführungsbeispiel genau drei Petrischalen 30 im Magazin 33 angeordnet. In einem ersten Schritt wird dann die automatische Handhabungseinrichtung 4, welche einen Greifer 40 mit einer ersten Greiferbacke 41 und einer zweiten Greiferbacke 42 aufweist, zum Sammelkopf 34 geführt und dieser von der Einrichtung 3 zur Luftüberwachung entnommen und auf einem Lagerplatz 5, welcher auf dem Magazin 3 angeordnet ist, abgelegt. Dieser Zustand ist in Figur 3 gezeigt. In einem nächsten Schritt wird der Greifer 40 zu einer der Petrischalen 30 im Magazin 33 geführt und eine der Petrischalen 30 wird aus dem Magazin entnommen. Die entnommene Petrischale wird auf eine Messstelle 6 der Einrichtung 3 zur Luftüberwachung aufgesetzt.

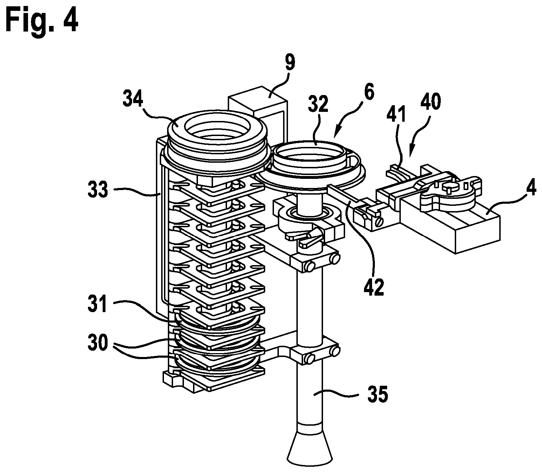

In einem nächsten Schritt nimmt der Greifer 40 dann den Deckel 31 der auf die Messstelle 6 platzierten Petrischale ab und legt den Deckel 31 im Magazin 33 ab. Dieser Zustand ist in Figur 4 gezeigt.

In einem nächsten Schritt nimmt dann der Greifer 40 den Sammelkopf 34 von der Lagerstelle 5 und positioniert diesen über die geöffnete Petrischale 30 an der Messstelle 6.

Nun kann der Messvorgang beginnen, indem die Einrichtung 3 zur Luftüberwachung Luft von oben durch den Sammelkopf 34 ansaugt und über das Luftaustrittsrohr 35 wieder abgibt. Wenn nun Keime im abgeschlossenen Raum 2 vorhanden sind, werden diese auf das Nährmedium in der unteren Aufnahme 31 der geöffneten Petrischale im Sammelkopf 34 zugeführt und können erfasst werden.

Nach einer vorbestimmten Zeitdauer muss die zur Überwachung eingesetzte Petrischale durch eine neue Petrischale ersetzt werden. Hierzu nimmt die automatische Handhabungseinrichtung 4 zuerst den Sammelkopf 34 wieder von der Einrichtung 3 zur Luftüberwachung ab und legt diesen auf dem Lagerplatz 5 ab. Anschließend wird der im Magazin 33 abgelegte Deckel 31 aus dem Magazin entnommen und auf die untere Aufnahme 31 der Petrischale an der Messstelle 6 aufgesetzt. Anschließend wird die so wieder geschlossene Petrischale 30 von der Messstelle 6 entnommen und im Magazin 33 abgelegt.

Falls eine weitere Messung ausgeführt werden soll, entnimmt die automatische Handhabungseinrichtung 4 dann eine nächste, unbenutzte Petrischale 30 aus dem Magazin 33 und positioniert diese wieder auf der Messstelle 6. Dann wiederholt sich der oben beschriebene Ablauf zur Luftüberwachung.

Die Verwendung des Magazins 33 hat ferner den Vorteil, dass eine Vielzahl von Petrischalen 30 in einem Schritt in und aus der Maschine gebracht werden können, wodurch die Handhabung weiter vereinfacht wird.

Somit kann über eine gesamte Charge eine mikrobiologische Luftüberwachung mit mehreren Petrischalen durchgeführt werden, wobei eine vollständig automatische Handhabung möglich ist.

Die automatische Handhabungseinrichtung 4 dieses Ausführungsbeispiels ist ferner dafür eingerichtet, dass die Behältnisse von der Füllstation 10 auch zur Verschlussstation 11 zugeführt werden. Dieser Vorgang wird durch die automatische Handhabungseinrichtung 4 in diesen Zeiträumen durchgeführt, in welchen kein Wechsel der Petrischale 30 zur Luftüberwachung notwendig ist. Somit weist die automatische Handhabungseinrichtung 4 neben dem Wechsel der Petrischalen noch zusätzliche Handhabungsaufgaben auf, nämlich eine Transportaufgabe von gefüllten Behältnissen von der Füllstation 10 zur Verschließstation 11. Es sei angemerkt, dass die automatische Handhabungseinrichtung 4 auch noch weitere Transportaufgaben übernehmen kann, beispielsweise das Zuführen von leeren Behältnissen zur Füllstation oder das Abführen von verschlossenen, gefüllten Behältnissen von der Verschließstation zu einer Lagerposition.

Somit kann gemäß der Erfindung eine mikrobiologische Luftüberwachung mit einem automatischen Petrischalenhandling erreicht werden. Dies ist im Vergleich mit den manuellen Handlingsvorgängen im Stand der Technik reproduzierbar und deutlich verlässlicher, da das Risiko des menschlichen Eingriffs bei der Erfindung nicht gegeben ist. Insbesondere im pharmazeutischen Bereich kann dadurch ein Risiko einer unerwünschten Kontamination, beispielsweise durch Partikel, die sich beim Bewegen der Handschuhe durch den Handschuheingriff von diesen ablösen können, oder durch ein ungeschicktes Petrischalenhandling, vermieden werden.

Insbesondere kann auch ein Wechseln der Petrischalen 30 während des Abfüllvorgangs nicht vergessen werden, da dies automatisiert durch eine Steuereinrichtung vorgenommen werden kann.

Auch kann durch die Identifikationseinrichtung 9 eine sichere und verfolgbare Dokumentation der Proben in den Petrischalen 30 erfolgen.

Es sei ferner angemerkt, dass der Greifer 40 des Roboters beispielsweise auch auswechselbar vorgesehen werden kann, falls gewisse Formatvorgaben der Behältnisse beispielsweise ein Greifen mit dem Greifer für die Petrischalen nicht ermöglichen würden. Hierzu kann eine automatische Greiferwechseleinrichtung im abgeschlossenen Raum 2 vorgesehen sein.

## Patentansprüche

1. Anlage zum Abfüllen und Verschließen von Medikamenten in Behältnisse, umfassend
- einen abgeschlossenen Raum (2), in welchem die Medikamente gehandhabt werden,
- eine Einrichtung (3) zur Überwachung der Luft im abgeschlossenen Raum (2) mit einer Messstelle (6) und wenigstens einer Petrischale (30), wobei die Petrischale (30) eine untere Aufnahme (31) und einen abnehmbaren Deckel (32) umfasst, und
- eine automatische Handhabungseinrichtung (4),
- wobei die automatische Handhabungseinrichtung (4) eingerichtet ist, automatisiert die Petrischale zur Messstelle (6) hin und zurück zu transportieren und den Deckel (32) von der unteren Aufnahme (31) der Petrischale (30) zu entfernen und wieder aufzusetzen,
**dadurch gekennzeichnet, dass**
die automatische Handhabungseinrichtung (4) eingerichtet ist, Transportschritte für Behältnisse zur Aufnahme der Medikamente auszuführen.

2. Anlage nach Anspruch 1, wobei die Einrichtung (3) zur Überwachung der Luft einen Luftkeimsammler mit einem Sammelkopf (34) und einem Magazin (33) zur Aufnahme einer Vielzahl von Petrischalen (30) umfasst.

3. Anlage nach Anspruch 2, wobei die automatische Handhabungseinrichtung (4) eingerichtet ist, den Sammelkopf (34) vom Luftkeimsammler abzunehmen und aufzusetzen.

4. Anlage nach einem der vorhergehenden Ansprüche, ferner umfassend eine Füllstation (10) zum Abfüllen der Medikamente in Behältnisse und eine Verschließstation (11) zum Verschließen der befüllten Behältnisse.

5. Anlage nach Anspruch 4, wobei die automatische Handhabungseinrichtung eingerichtet ist, die Behältnisse von der Füllstation (10) zur Verschließstation (11) zu transportieren.

6. Anlage nach einem der vorhergehenden Ansprüche, wobei die automatische Handhabungseinrichtung (4) einen Roboter, insbesondere einen 6-Achs-Roboter, umfasst.

7. Anlage nach Anspruch 6, wobei der Roboter einen Greifer (40) mit einer ersten und zweiten Greiferbacke (41, 42) umfasst.

8. Anlage nach einem der vorhergehenden Ansprüche, ferner umfassend eine Identifikationseinrichtung (9), welche eingerichtet ist, Petrischalen (30) zu identifizieren und eine vorbestimmte Messzeit zu einer identifizierten Petrischale (30) zuzuordnen.

9. Anlage nach Anspruch 8, wobei jede Petrischale (30) ein individuelles, optisches Erkennungszeichen aufweist und die Identifikationseinrichtung (9) eine optische Erkennungseinrichtung zum Erkennen des Erkennungszeichens der Petrischale aufweist.

10. Anlage nach einem der vorhergehenden Ansprüche, wobei die Anlage eine Isolatoranlage oder eine RABS-Anlage ist.

## Claims

1. System for filling and sealing medicaments in containers, comprising
- an enclosed space (2) in which the medicaments are handled,
- a device (3) for monitoring the air in the enclosed space (2) with a measuring point (6) and at least one Petri dish (30), wherein the Petri dish (30) comprises a lower receptacle (31) and a removable lid (32), and
- an automatic handling device (4),
- wherein the automatic handling device (4) is designed to automatically transport the Petri dish to the measuring point (6) and back and to remove the lid (32) from the lower receptacle (31) of the Petri dish (30) and put it back on,
**characterized in that**
the automatic handling device (4) is designed to carry out transport steps for containers for receiving the medicaments.

2. System according to claim 1, wherein the device (3) for monitoring the air comprises an air sampler having a collecting head (34) and a magazine (33) for receiving a large number of Petri dishes (30).

3. System according to claim 2, wherein the automatic handling device (4) is designed to remove the collecting head (34) from the air sampler and put it on.

4. System according to any of the preceding claims, further comprising a filling station (10) for filling the medicaments into containers and a sealing station (11) for sealing the filled containers.

5. System according to claim 4, wherein the automatic handling device is designed to transport the containers from the filling station (10) to the sealing station (11).

6. System according to any of the preceding claims, wherein the automatic handling device (4) comprises a robot, in particular a 6-axis robot.

7. System according to claim 6, wherein the robot comprises a gripper (40) having a first and second gripping jaw (41, 42).

8. System according to any of the preceding claims, further comprising an identification device (9) which is designed to identify Petri dishes (30) and to assign a predetermined measuring time to an identified Petri dish (30).

9. System according to claim 8, wherein each Petri dish (30) has an individual, optical identification mark and the identification device (9) has an optical recognition device for recognizing the identification mark of the Petri dish.

10. System according to any of the preceding claims, wherein the system is an isolator system or an RABS system.

## Revendications

1. Installation pour le remplissage et la fermeture de récipients avec des médicaments, comprenant
- un espace fermé (2) dans lequel les médicaments sont manipulés,
- un dispositif (3) pour la surveillance de l'air dans l'espace fermé (2) comportant un emplacement de mesure (6) et au moins une boîte de Pétri (30), dans laquelle la boîte de Pétri (30) comprend un logement inférieur (31) et un couvercle (32) amovible, et
- un dispositif de manipulation automatique (4),
- dans laquelle le dispositif de manipulation automatique (4) est conçu pour transporter de manière automatisée la boîte de Pétri vers l'emplacement de mesure (6) et à l'écart de celui-ci, et pour retirer le couvercle (32) du logement inférieur (31) de la boîte de Pétri (30) et le remettre en place,
**caractérisée en ce que**
le dispositif de manipulation automatique (4) est configuré pour exécuter des étapes de transport pour des récipients destinés à recevoir les médicaments.

2. Installation selon la revendication 1, dans laquelle le dispositif (3) pour la surveillance de l'air comprend un collecteur de germes dans l'air comportant une tête collectrice (34) et un magasin (33) destiné à recevoir une pluralité de boîtes de Pétri (30).

3. Installation selon la revendication 2, dans laquelle le dispositif de manipulation automatique (4) est conçu pour retirer la tête collectrice (34) du collecteur de germes dans l'air et la mettre en place.

4. Installation selon l'une des revendications précédentes, comprenant en outre un poste de remplissage (10) permettant de remplir des récipients avec des médicaments et un poste de fermeture (11) permettant de fermer les récipients remplis.

5. Installation selon la revendication 4, dans laquelle le dispositif de manipulation automatique est conçu pour transporter les récipients depuis le poste de remplissage (10) jusqu'au poste de fermeture (11).

6. Installation selon l'une des revendications précédentes, dans laquelle le dispositif de manipulation automatique (4) comprend un robot, en particulier un robot 6 axes.

7. Installation selon la revendication 6, dans laquelle le robot comprend un moyen de préhension (40) comportant une première et une seconde mâchoire de préhension (41, 42).

8. Installation selon l'une des revendications précédentes, comprenant en outre un dispositif d'identification (9) qui est configuré pour identifier des boîtes de Pétri (30) et pour attribuer un temps de mesure prédéterminé à une boîte de Pétri (30) identifiée.

9. Installation selon la revendication 8, dans laquelle chaque boîte de Pétri (30) présente un marquage de reconnaissance optique individuel et le dispositif d'identification (9) présente un dispositif de reconnaissance optique permettant de reconnaître le marquage de reconnaissance de la boîte de Pétri.

10. Installation selon l'une des revendications précédentes, dans laquelle l'installation est une installation à isolateur ou une installation de type RABS.
